# EUROPEAN PATENT SPECIFICATION

(11) **EP 1 107 982 B1**
(45) Date of publication and mention of the grant of the patent: **19.05.2004**
(21) Application number: 99943807.0
(22) Date of filing: 18.08.1999
(51) Int. Cl.: C07K 7/56, A61K 38/12, C07K 1/14, A61P 31/10

(54) **REVERSIBLE BORON COMPLEXES OF 1,2-(CIS)-DIOL CYCLIC PEPTIDES**
REVERSIBLE BORKOMPLEXE VON 1,2-(CIS)-DIOL ZYKLISCHEN PEPTIDEN
COMPLEXES REVERSIBLES DU BORE DE PEPTIDES CYCLIQUES CONTENANT DES GROUPES 1,2-(CIS)-DIOL

(30) Priority: 28.08.1998 US 98267 P
(43) Date of publication of application: 20.06.2001
(73) Proprietor: Eli Lilly & Company, Indianapolis, Indiana 46285 (US)
(72) Inventor: MOSER, Brian, Allen, Indianapolis, IN 46217 (US); BAKER, Jeffrey, Clayton, Indianapolis, IN 46205 (US)
(74) Representative: Benson, John Everett
(86) International application number: PCT/US1999/019066
(87) International publication number: WO 2000/012540

(56) References cited:
- WO-A-97/47645
- US-A- 5 631 364
- JAMISON E.A.: "The synthesis and antifungal activity of nitrogen containing hemiaminal ethers of LY303366" JOURNAL OF ANTIBIOTICS., vol. 51, no. 2, February 1998 (1998-02), pages 239-242, XP002126600 JAPAN ANTIBIOTICS RESEARCH ASSOCIATION. TOKYO., JP ISSN: 0021-8820

## Description

The present invention relates to the formation of reversible borate or boronate complexes of 1,2-*cis*-diol cyclic-peptides and their use as a means for purification, isolation, stabilization andior water solubilization of their respective parent 1,2-*cis*-diol cyclic-peptide, in particular, Echinocandin compounds and derivatives thereof. The invention also relates to pharmaceutical formulations based on the reversible borate or boronate complexes described above.

Macromolecules and in particular cyclic peptides such as those related to the antifungal agent Echinocandin B (ECB) tend to be very hydrophobic. Consequently, large volumes of organic solvents are generally required to maintain solubility and to enable commercial-scale purification. The use of organic solvents raises several toxicological, environmental and regulatory concerns. For example, volatile organic compounds (VOCs) are subject to air quality regulation and organic solvents generally require special handling and disposal. If the active compound is very insoluble in water, then pharmaceutical formulations for internal consumption are generally restricted to formulations as dry powders or solids thus limiting the means by which the medicament can be administered to the patient. For example, water insoluble compounds typically are not used in intravenous (IV) solutions. Therefore, there is a need for a process whereby hydrophobic compounds, such as Echinocandin B, can be selectively made more hydrophilic or aqueous-soluble to reduce or eliminate the need for organic solvents during the manufacturing process and allows one to easily revert the compounds back to their original state at the end of the process. In addition, there is a need for a method whereby the water-solubility of hydrophobic pharmaceutically active compounds can be increased so that water-based formulations may be used in medicaments without adversely affecting the potency of the drug.

Boronates have been used to purify nucleic acids, carbohydrates and glycoproteins by means of immobilized affinity chromatography. For example, ribonucleosides have been shown to bind via the *cis*-hydroxyl groups of the ribose to boronate ligands that are attached to an insoluble resin at pH values above the pKa of the boronate. When the pH is lowered, the boronate reverts from a tetrahedral to trigonal planar form and the ribonucleoside is released from the resin. See, i.e., Liu, X.C., et al., *J. of Chromatography A*, **687**, 61-69 (1994); Alvarez-Gonzales, R., et al., *Anal. Biochem.,* **135**, 69-77 (1983); Weith, H.L., et al., *Biochemistry*, **9**, 4396-4401 (1970); and Scouten, W.H., *Solid Phase Biochemistry*, Wiley, New York, p 149 (1983). The purification process requires dissolving or suspending the cis-diol in an alkaline solution which is generally problematic for cyclic peptides due to opening of the ring nucleus. This can be particularly troublesome in a manufacturing process where the process times are lengthy resulting in an increased opportunity for degradation.

WO 97/47645 discloses a process for preparing certain aza cyclohexapeptides which includes a step of reacting a cyclic hexapeptide containing 1,2-*cis*-diol moieties with phenylboronic acid, *p*-methoxyphenylboronic acid or methaneboronic acid to afford an intermediate boronate adduct.

Jamison, J.A., et al., *Journal of Antibiotics*, vol. 51, no. 2, 239-242 (1998), discloses the synthesis and anti-fungal activity of certain nitrogen-containing hemiaminal ethers of a cyclic hexapeptide referred to as LY303366.

The present invention provides a reversible cyclic peptide adduct comprising a boric or boronic acid complexed with a cyclic Echinocandin anti-fungal peptide having at least one 1,2-*cis*-diol moiety wherein said adduct is ionic in character and has a tetrahedral geometry at the boron atom and is more water-soluble than said cyclic peptide having at least one 1,2-*cis*-diol moiety. Surprisingly, the boronate adducts of 1,2-*cis*-diol cyclic peptides having the following general structure are significantly more soluble in aqueous solutions than their corresponding parent *cis*-diol, cyclic-peptide compound. 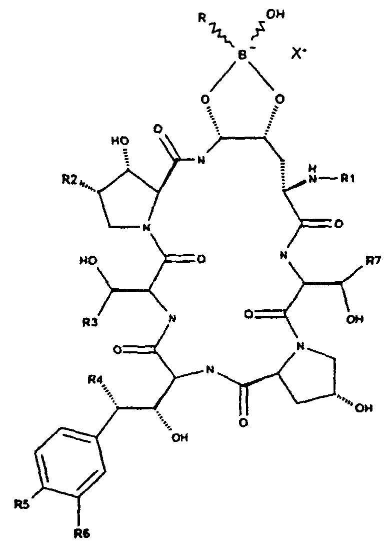 wherein R is a hydroxy, an alkoxy group, a phenoxy group, an alkyl group, a phenyl group (e.g., *m*-aminophenyl or *p*-carboxyphenyl), a thiol, a thioalkyl group, or a thiophenyl group; R¹ is -H or -C(O)R^{1a} where R^{1a} is an alkyl group, an alkenyl group, an alkynyl group, an aryl group, heteroaryl group or combinations thereof; R² is -H or -CH₃; R³ is -H,-CH₃, -CH₂CONH₂ or -CH₂CH₂NH₂; R⁴ is -H or -OH; R⁵ is -OH, -OPO₃H₂, or -OSO₃H; R⁶ is -H or -OSO₃H and X⁺ is any suitable cation (e.g., H⁺, cation of an alkali metal, NH₄⁺, etc. including any pharmaceutically acceptable cations).

The term "aqueous" refers to water as well as homogeneous mixtures of water with organic solvents such as, methanol, ethanol, propanol, acetonitrile, dimethylformamide, dimethylsulfoxide, propylene glycol, polyethylene glycols (e.g., PEG400, PEG300), and the like. Generally, the organic solvent content of the homogeneous mixture is less than 50% by weight, preferably less than 25%, more preferably less than 10%, most preferably 0%.

In another embodiment of the present invention, a method is provided for forming the reversible cyclic peptide adduct of the invention, comprising the steps of
(i) providing an aqueous solution of a boric or boronic acid,
(ii) adding a cyclic Echinocandin anti-fungal peptide compound having at least one 1,2-*cis*-diol moiety to said aqueous solution, and
(iii) adjusting the pH of said aqueous solution to a value sufficient to effect complexation between said boric or boronic acid and said cyclic Echinocandin anti-fungal peptide compound under alkaline conditions.

As used herein, the terms "complex", "complexed" and "complexation" refer to the reaction between the boric or boronic acid and the 1,2-*cis*-diol moiety of the cyclic peptide compound to form the reversible adduct described above. The borate/*cis*-diol adduct(1) shown below in the detailed description provides an additional illustration of a complex with boric acid in a more generic form.

Correspondingly, the terms "decomplex", "decomplexed" and "decomplexation" refer to the reverse reaction where the adduct reverts back to its original components (i.e., the boric or boronic acid and the 1,2-*cis*-diol, cyclic peptide).

In yet another embodiment of the present invention, a method is provided for purifying a cyclic Echinocandin anti-fungal peptide having a 1,2-*cis*-diol moiety, comprising in the following order the steps of
(i) providing a crude mixture of a cyclic Echinocandin anti-fungal peptide having at least one 1,2-*cis*-diol functionality,
(ii) complexing said at least one 1,2-*cis*-diol functionality of said cyclic Echinocandin anti-fungal peptide with a boric or boronic acid to form a reversible adduct of the invention,
(iii) solubilizing said reversible adduct in an aqueous solution,
(iv) either
   (a) removing any insoluble materials from said aqueous solution or
   (b) concentrating said aqueous solution to form a concentrate, absorbing said concentrate onto a reverse-phase hydrophobic resin packed in a chromatography column, eluting with an aqueous solvent system, and combining effluent fractions containing said reversible adduct into a single effluent solution,
(v) acidifying said aqueous solution to a pH value equal to or less than the pKa of said boric or boronic acid to decomplex said reversible adduct, and
(vi) recovering said cyclic Echinocandin anti-fungal peptide from said aqueous solution.

In yet another embodiment, a pharmaceutical formulation is provided comprising the reversible adduct described above. The adduct may be used in a method for treatment of a host in need of treatment for a fungal infection which includes administration of a therapeutic amount of the reversible borate or boronate/Echinocandin adduct (described above) to a host and decomplexing the adduct to release the pharmaceutically active 1,2-*cis*-diol, cyclic peptide.

It is believed that boric and boronic acids behave in a Lewis acid fashion where they accept a hydroxyl group under alkaline conditions and assume a tetrahedral geometry as depicted below for boric acid.

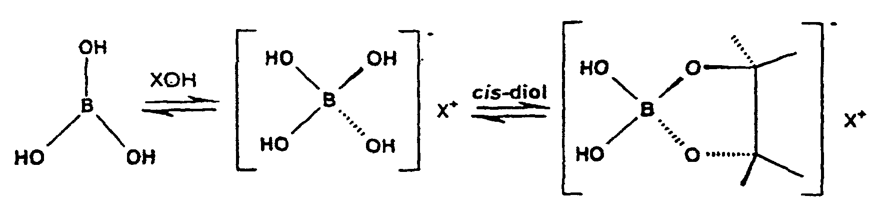

### borate/cis-diol adduct(1)

Unlike the trigonal planar geometry of boric acid in neutral or acidic conditions, the tetrahedral geometry in alkaline conditions enables coupling to 1,2-*cis*-diols to form a borate/*cis*-diol adduct(1). By changing the acidity of the solution the adduct becomes reversible. In other words, a borate/diol adduct forms under alkaline conditions and reverts back to the separate starting materials when subjected to acidic conditions. The same is also true for adducts formed with boronic acids.

Since the borate and boronate adducts are ionic in character, the hydrophilicity or water-solubility of the complexed material is enhanced. Hence, materials containing a 1,2-*cis*-diol moiety that are generally insoluble or only slightly soluble in aqueous solutions can be made soluble or more soluble in aqueous solutions by forming a borate or boronate adduct. Surprisingly. applicants observed a significant change in water solubility when 1,2-*cis*-diol cyclic peptides are complexed with boric (or boronic) acid. For example, Example 1 below shows a 10-fold increase in solubility of an Echinocandin B derivative in the presence of *m*-aminophenyl boronic acid at a pH of 9.0 in an aqueous solution. At concentrations of 30 mg/ml, the aqueous solution of the non-complexed Echinocandin B derivative is an opaque slurry; whereas, the aqueous solution of the boronate complex is a transparent solution. The increased solubility in aqueous solutions provides several advantages. It reduces the amount of organic solvents required in the manufacturing process which decreases costs and regulatory concerns. The process not only reduces the probability of residual solvent in a pharmaceutically active material which may be detrimental to the overall toxicology of a resultant medicament; it also allows one to design water-based pharmaceutical formulations based on the boronate adduct. Due to the ease by which the boronate complex is formed and the increased solubility of the complex, the process also provides a convenient method for assaying samples containing 1,2-*cis*-diol, cyclic-peptides.

Unlike the parent 1,2-*cis*-diol, cyclic-peptides, the corresponding borate (or boronate) adducts are more stable at high pHs. Cyclic peptides containing an omithine unit are known to lose the omithine unit under alkaline conditions due to the hydroxyl groups. The boronate adduct provides a means of protecting the omithine unit from fragmentation by complexing with the 1,2-*cis*-diol moiety. Once the alkaline reaction is completed, the boronate functionality can be easily removed by simply reducing the pH of the solution (or mixture).

Any Echinocandin-type natural product or semi-synthetic derivative may be used to form a borate (or boronate) adduct so long as the *cis*-hydroxyl groups of the ornithine peptide unit are present and not blocked. The ornithine α-amino group may be acylated or non-acylated. As used herein, the term "Echinocandin anti-fungal peptide" refers to anti-fungal peptides having the following general structure including any simple derivatives thereof: 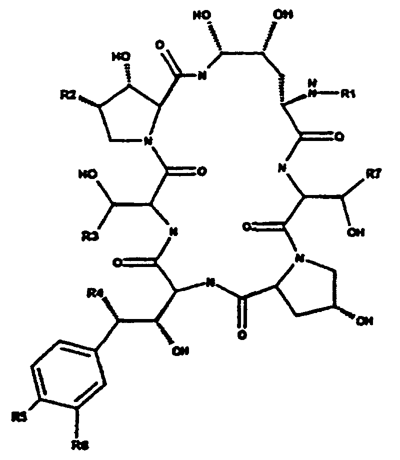 wherein R¹ is -H or -C(O)R where R is an alkyl group, an alkenyl group, an alkynyl group, an aryl group, or heteroaryl group; R² is -H or -CH₃; R³ is -H, -CH₃, -CH₂CONH₂ or -CH₂CH₂NH₂; R⁴ is -H or -OH; R⁵ is -OH, -OPO₃H₂, or -OSO₃H; and R⁶ is -H or -OSO₃H. Pharmaceutically acceptable salts, esters and hydrates are also included.

The term "alkyl" refers to a hydrocarbon radical of the general formula CₙH₂ₙ₊₁ containing from 1 to 30 carbon atoms unless otherwise indicated. The alkane radical may be straight, branched, cyclic, or multi-cyclic. The alkane radical may be substituted or unsubstituted. Similarly, the alkyl portion of an alkoxy group or alkanoate have the same definition as above.

The term "alkenyl" refers to an acyclic hydrocarbon containing at least one carbon-carbon double bond. The alkene radical may be straight, branched, cyclic, or multi-cyclic. The alkene radical may be substituted or unsubstituted.

The term "alkynyl" refers to an acyclic hydrocarbon containing at least one carbon-carbon triple bond. The alkyne radical may be straight, or branched. The alkyne radical may be substituted or unsubstituted.

The term "aryl" refers to aromatic moieties having single (e.g., phenyl) or fused ring systems (e.g., naphthalene, anthracene, phenanthrene, etc.). The aryl groups may be substituted or unsubstituted. Substituted aryl groups include a chain of aromatic moieties (e.g., biphenyl, terphenyl, phenylnaphthalyl, etc.)

The term "heteroaryl" refers to aromatic moieties containing at least one heteratom within the aromatic ring system (e.g., pyrrole, pyridine, indole, thiophene, furan, benzofuran, imidazole, pyrimidine, purine, benzimidazole, quinoline, etc.). The aromatic moiety may consist of a single or fused ring system. The heteroaryl groups may be substituted or unsubstituted.

In the present invention, for example, the term alkyl group allows for substituents which is a classic alkyl, such as methyl, ethyl, propyl, hexyl, isooctyl, dodecyl, stearyl, etc. The term group specifically envisions and allows for substitutions on alkyls which are common in the art, such as hydroxy, halogen, alkoxy, carbonyl, kelo, ester, carbamato, etc., as well as including the unsubstituted alkyl moiety. However, it is generally understood by those skilled in the art that the substituents should be selected so as to not adversely affect the pharmacological characteristics of the compound or adversely interfere with the use of a medicament containing the compound. Suitable substituents for any of the groups defined above include alkyl, alkenyl, alkynyl, aryl, halo, hydroxy, alkoxy, aryloxy, mercapto, alkylthio, arylthio, mono- and dialkyl amino, quaternary ammonium salts, aminoalkoxy, hydroxyalkylamino, aminoalkylthio, carbamyl, carbonyl, carboxy, glycolyl, glycyl, hydrazino, guanyl, and combinations thereof.

The term "natural product" refers to those secondary metabolites, usually of relatively complex structure, which are of more restricted distribution and more characteristic of a specific source in nature. Suitable natural product starting materials belonging to the Echinocandin cyclic peptide family include Echinocandin B, Echinocandin C, Aculeacin Ay, Mulundocandin, Sporiofungin A, Pneumocandin A₀, WF11899A, and Pneumocandin B₀.

Semi-synthetic cyclic peptides are generally prepared by deacylating the naturally occurring cyclic peptides using procedures known in the art to provide the corresponding amino nucleus (where R¹ is hydrogen). This reaction is typically carried out enzymatically, by exposing the naturally occurring cyclic peptide to a deacylase enzyme. The deacylase enzyme may be obtained from the microorganism *Actinoplanes utahensis* and used substantially as described in U.S. Patent Nos. 4,293,482 and 4,304,716. The deacylase enzyme may also be obtained from the *Pseudomonas* species. Deacylation may be accomplished using whole cells of *Actinoplanes utahensis* or *Pseudomonas*, the crude or purified enzyme thereof, or using an immobilized form of the enzyme, (see. i.e., EP 460882)

The amino nucleus (R¹ = H) is re-acylated using procedures known in the art to provide an Echinocandin-type compound where R¹ is an acyl group represented by -C(O)R. Acylation of the amino group may be accomplished in a variety of ways well known to those skilled in the art. For example, the amino group may be acylated by reaction with an appropriately substituted acyl halide, preferably in the presence of an acid scavenger such as a tertiary amine (e.g., triethylamine). The reaction is typically carried out at a temperature between about -20°C to 25°C. Suitable reaction solvents include polar aprotic solvents, such as dioxane or dimethylformamide. Solvent choice is not critical so long as the solvent employed is inert to the ongoing reaction and the reactants are sufficiently solubilized to effect the desired reaction.

The amino group may also be acylated by reaction with an appropriately substituted carboxylic acid, in the presence of a coupling agent. Suitable coupling agents include dicyclohexylcarbodiimide (DCC), N,N'-carbonyldiimidazole, bis(2-oxo-3-oxazolidinyl)phosphinic chloride (BOP-Cl), N-ethoxycarbonyl-2-ethoxy-1,2-dihydroquinoline (EEDQ), benzotriazole-1-yloxytripyrrolidinophosphoniurn hexafluorophosphate (PyBOP) and the like.

Alternately, the amino group may be acylated with an activated ester of a carboxylic acid such as p-nitrophenyl, 2,4,5-trichlorophenyl, hydroxybenzotriazole hydrate (HOBT·H₂O), pentafluorophenol, and N-hydroxysuccinimide carboxylate esters. Preferred acylating moieties are the 2,4,5-trichlorophenyl and HOBT carboxylate esters. The reaction is typically run 1 to 65 hours at a temperature from about 0°C to 30°C in an aprotic solvent. The reaction is generally complete after about 24 to 48 hours when carried out at a temperature between about 15°C to 30°C. Suitable solvents include tetrahydrofuran and dimethylformamide or mixtures thereof. The amino group is generally present in equimolar proportions relative to the activated ester or with a slight excess of the amino group.

A borate or boronate adduct may be formed with any of the foregoing Echinocandin-type compounds or synthetic intermediates that contain a 1,2-*cis*-diol moiety. The adduct is formed by simply mixing the boric acid with the cyclic peptide materials under basic conditions. The order in which the reagents are added is not particularly important. However, to minimize potential for fragmentation of the cyclic peptide ring, preferably the boric acid is added to the cyclic peptide prior to increasing the pH of the mixture. Like the order of addition, the temperature at which the reaction is operated also is not particularly important; however, to minimize potential ring fragmentation, preferably the reaction is run near or below room temperature (≤ 20°C).

The pH may be increased with any base reagent capable of acting as a Lewis base with respect to the boric acid. The particular base used is not critical. Suitable bases include sodium hydroxide, ammonium hydroxide, ammonium bicarbonate, potassium carbonate, potassium hydroxide and mixtures thereof. The amount of base added will vary depending upon the particular boric acid or boronic acid used. Generally, the amount of base added is equal to the concentration needed to achieve a pH value sufficient to effect optimum complexation of the boric acid with the 1,2-*cis*-diol functionality. For most reactions, the solution is adjusted to a pH between 7.5 and 9.5.

Suitable boric and boronic acid reagents include boric acid, alkylboronic acids (e.g., ethylboronic acid, propylboronic acid, butylboronic acid, etc.), heterocycloalkyl boronic acids (e.g., tetrahydrofuranylboronic acid). arylboronic acids (e.g., phenylboronic acid, o-methylphenyl-boronic acid, *m*-aminophenylboronic acid, *p*-methylphenylboronic acid, *p*-carboxyphenylboronic acid, [*o*-(diisopropylamino)carbonyl]phenylboronic acid, *o*-formylphenylbaronic acid, *m*-formylphenylboronic acid, *p*-methoxyphenylboronic acid, *p*-nitrophenylboronic acid, *p*-fluorophenylboronic acid, *p*-bromophenylboronic acid, *p*-trifluoromethylphenylboronic acid, 4,4'-diphenyldiboronic acid, 1-naphthylboronic acid, etc.), heteroarylboronic acids (e.g., thiophene-2-boronic acid, thiophene-3-boronic acid, 2-formylthiophene-2-boronic acid, 5-chlorothiophene-2-boronic acid. 5-acetylthiophene-2-boronic acid, benzo[b]thiophene-2-boronic acid, benzo[b]furan-2-boronic acid, indole-5-boronic acid, etc.), and the like, *m*-Aminophenylboronic acid is particularly preferred when complexed with the Echinocandin compound C1 described below in the Examples. Preferably, the substituents on the boronic acid are chosen such that they do not sterically hinder the formation of the tetrahedral configuration so that optimum complexation may occur. Preferably, the boric or boronic acid is chosen such that the optimum pH to achieve complexation is less than 9.0 to reduce the potential for fragmentation of the cyclic-peptide ring. Suitable boronic acids are available from a variety of commercial sources such as TCI America (Portland, OR), Aldrich Chemical (Milwaukee, WI), Lancaster Synthesis (Windham, NH), or alternatively, synthesized using methods described in Beesley et al., *Biochem. J*., **209**, 229-233 (1983) or Matteson, *Acc. Chem. Res*., **21**, 294-300 (1988).

Reversion of the borate (or boronate) adduct back to the original 1,2-*cis*-diol, cyclic-peptide can be accomplished by acidifying the solution containing the borate adduct with any suitable acid to lower the pH. The choice of acid is not particularly important so long as the pH can be lowered to a value sufficient to remove the borate from the *cis*-diol moiety. The optimum pH value will depend on the pKa of the particular boric acid used for complexation. Suitable acids include hydrochloric acid, acetic acid, phosphoric acid. sulfuric acid, and the like.

It will be understood by those skilled in the art that the formation of the borate or boronate adduct provides a useful means of changing the hydrophilicity of any 1,2-*cis*-diol intermediate or final product. Increasing the hydrophilicity of the 1,2-*cis*-diol material not only decreases the amount of organic solvents needed in the process, but may also assist in the isolation and separation of the 1,2-*cis*-diol material from other non-1,2-*cis*-diol containing materials at any stage of a synthetic process.

The boronate adducts may be used in a variety of purification methods. For example, the increased solubility of the adduct allows one to separate the soluble adduct from other insoluble materials. An example of a simple purification process based on solubility differences would include the following steps: (i) providing a crude mixture of a 1,2-*cis*-diol cyclic peptide; (ii) complexing the 1,2-cis-diol moiety of the cyclic-peptide with a boric (or boronic) acid to form a borate (or boronate) adduct; (iii) solubilizing the borate adduct in an aqueous solution, (iv) removing any insoluble materials from the aqueous solution, (v) acidifying the aqueous solution to a pH value that is equal to or less than the pKa of the boric (or boronic) acid; and (vi) recovering the 1,2-*cis*-diol cyclic-peptide from the acidified solution. The removal of insoluble materials in step (iv) can be accomplished by simple filtration or centrifugation followed by decanting of the liquid. Alternatively, the aqueous solution from step (iii) can be concentrated and separated on a reverse-phase chromatography column (high pressure or preparation column) packed with a hydrophobic reverse phase resin (e.g., styrene/divinylbentene resin) and eluted with an aqueous solvent system (e.g., acetonitrile/water, acetic add/water, etc.).

The fractions from the chromatographic effluents containing the boronate adduct would then be combined and acidified to decomplex the adduct into its original components (boronic acid and the 1,2-*cis*-diol, cyclic-peptide). The purified cyclic-peptide product can then be recovered using conventional methods such as, filtration, crystallization, solvent extraction followed by evaporative concentration, reverse osmosis, lyophilization, etc.

Theoretically, an anion exchange column would work as well; however, initial studies have shown that the Echinocandin C1/m-APBA adduct (see Example 1) binds too tightly to Q-Sepharose (registered trade mark) Fast Flow resin and can be removed only by eluting with a solvent system having sufficient acidity to decomplex the boronate adduct. None the less, the boronate adduct has been shown to bind to an anion exchange resin; therefore, anion exchange chromatography would be a viable method for purification. Those skilled in the art would know how to select the proper anion exchange resin to optimize the system.

A reverse purification process is also possible. For example, if one wanted to remove a cyclic peptide impurity that contained a 1,2-*cis*-diol moiety from a desired compound that did not contain a 1,2-*cis*-diol moiety, then one could use the same process described above to remove the unwanted 1,2-*cis*-diol, cyclic peptide.

The water-solubility and reversibility of the boronate adduct makes it attractive for use in aqueous based pharmaceutical formulations, in particular, aqueous intravenous (IV) solutions. The boronate adduct would remain soluble in the water-based medicament during administration to the patient. The active cyclic-peptide material would then be released from the boronate upon subjection to acidic conditions in the body. Of course, the reversibility of the boronate would occur within the body regardless of the means by which the drug is administered to the patient. Therefore, the boronate adduct is not necessarily restricted to aqueous-based formulations. Other nonaqueous-based medicaments for administration to the patient could be used as well (e.g., capsules, suppositories, powders, etc.).

A typical formulation comprises the boronate/1,2-*cis*-diol, cyclic-peptide adduct (or its pharmaceutically acceptable salt, ester or hydrate) in combination with a pharmaceutically acceptable carrier, diluent or excipient. The adduct is typically formulated into pharmaceutical dosage forms to provide an easily controllable dosage of the drug and to give the patient an elegant and easily handleable product. Formulations may comprise from 0.1% to 99.9% by weight of the adduct, more generally from about 10% to about 30% by weight.

As used herein, the term "unit dose" or "unit dosage" refers to physically discrete units that contain a predetermined quantity of active ingredient calculated to produce a desired therapeutic effect. When a unit dose is administered orally or parenterally, it is typically provided in the form of a tablet, capsule, pill, powder packet, topical composition, suppository, wafer, measured units in ampoules or in multidose containers, etc. Alternatively, a unit dose may be administered in the form of a dry or liquid aerosol which may be inhaled or sprayed.

The dosage to be administered may vary depending upon the physical characteristics of the patient, the severity of the patient's symptoms, and the means used to administer the drug. The specific dose for a given patient is usually set by the judgment of the attending physician.

Suitable carriers, diluents and excipients are well known to those skilled in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which the active ingredient is being applied. The formulations may also include wetting agents, lubricating agents, emulsifiers, suspending agents, preservatives, sweeteners, stabilizers, perfuming agents, flavoring agents and combinations thereof.

Echinocandin-type compounds have been shown to exhibit antifungal and antiparasitic activity such as growth inhibition of various infectious fungi including Candida spp. (i.e.. *C. Albicans, C. Parapsilosis*, *C. Krusei, C. Glabrata, C. Tropicalis*, or *C. Lusitaniaw*); Torulopus spp.(i.e., *T. Glabrata*); Aspergillus spp. (i.e., *A. Fumigatus*); Histoplasma spp. (i.e., *H. Capsulatum*), Cryptococcus spp. (i.e., *C. Neoformans*); Blastomyces spp. (i.e., *B. Dermatitidis*); Fusarium spp.; Trichophyton spp., *Pseudallescheria boydii, Coccidioides immits, Sporothrix schenckii*, etc.

Compounds of this type also inhibit the growth of certain organisms primarily responsible for opportunistic infections in immunosuppressed individuals, such as growth inhibition of *Pneumocystis carinii* (the causative organism of pneumocystis pneumonia PCP in AIDS and other immunocompromised patients). Other protozoans that are inhibited by Echinocandin-type compounds include Plasmodium spp., Leishmania spp., Trypanosoma spp., Cryptosporidium spp., Isospora spp., Cyclospora spp., Trichomnas spp., Microsporidiosis spp., etc.

U.S. Patent Nos. 4,293,489; 4,320,052; 5,166,135; and 5,541,160 describe a variety of N-acyl derivatized Echinocandin compounds that provide varying degrees of antifungal and antiprotozoal activities. Additional examples of active N-acyl derivatized Echinocandin compounds may be found in EP 359529; 448353; 447186; 462531; and 561639.

A host in need of treatment for a fungal infection may be treated by administering an effective amount of an Echinocandin/borate adduct (or a pharmaceutically acceptable salt, ester or hydrate thereof). Once the Echinocandin/borate adduct is subjected to acidic conditions in the host, the borate adduct decomplexes or reverts back to the original active Echinocandin compound and the corresponding boric acid. For example, the gastric acids in the stomach are more than sufficient to complete the reversion.

A "host" refers to an organism in or on which a parasite lives, deriving its body substance or energy from the host.

A preferred method includes treating a *Candida albicans* or *Aspergillus fumigatis* infection. The term "effective amount" refers to an amount of active compound which is capable of inhibiting fungal activity. The dose administered will vary depending on such factors as the nature and severity of the infection, the age and general health of the host and the tolerance of the host to the antifungal agent. The particular dose regimen likewise may vary according to these factors. The medicament may be given in a single daily dose or in multiple doses during the day. The regimen may last from about 2-3 days to about 2-3 weeks or longer. A typical daily dose (administered in single or divided doses) contains a dosage level between about 0.01 mg/kg to 100 mg/kg of body weight of an active compound. Preferred daily doses are generally between about 0.1 mg/kg to 60 mg/kg and more preferably between about 2.5 mg/kg to 40 mg/kg.

### EXAMPLES

Unless indicated otherwise, all chemicals can be acquired from commercial sources (i.e., Aldrich Chemical, Sigma, etc.) in reagent grade or better. Centrifugation was performed using an IEC benchtop clinical centrifuge equipped with six-place swinging-bucket rotor (for samples > 1.5 ml) and an Eppendorf tabletop centri fuge model 5415C equipped with standard 18-place rotor (for samples ≤ 1.5 ml). HPLC analyses were performed using a Hewlett Packard Liquid Chromatograph Model 1090 equipped with a 0.46 x 25 cm Zorbax™ SB-C8 column. All injections were 10 µl, followed by gradient elution from 40-100% acetonitrile (AcN) over 25 minutes and UV detection at 280 nm. Unless otherwise stated, samples were diluted in 0.25 N acetic acid/AcN prior to injection to facilitate decomplexation and ensure solubility in the HPLC solvent system. All alkaline pH adjustments were made by addition of a 1:1 mixture of ammonium hydroxide and 50% sodium hydroxide. Measurements were taken with a Beckman φ40 pH meter calibrated with pH 7 and pH 10 standards.

The following examples illustrate the enhanced water solubility and chemical stability of the following Compound C1 in the presence of *m*-aminophenylboronic acid (*m*-APBA). Compound C1 may be synthesized according to the procedures described in EP 561639.

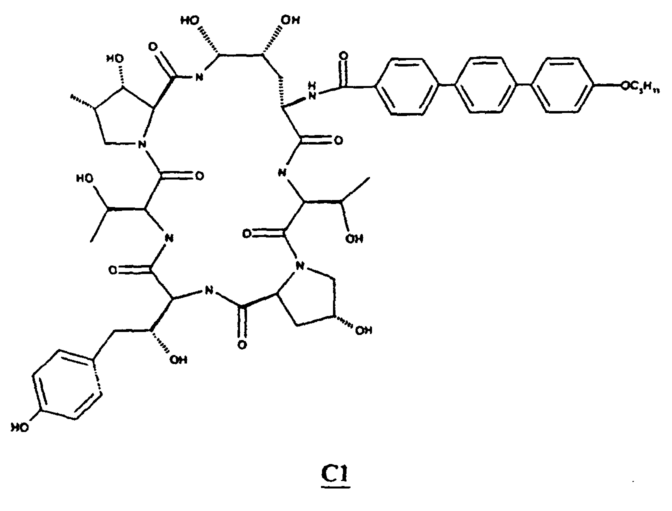

### Example 1

### Solubility of Compound C1 in the presence of m-APBA

Crude C1 was suspended at 50 mg/ml (65% potency: 32.5 mg/ml, or 28.5 mM) in 4 ml of either 100 mM *m*-APBA or 100 mM ammonium bicarbonate (control) at pH 5.0 and pH 9.0. The samples were stirred at room temperature for 2 hours, at which time aliquots were removed and assayed both before and after centrifugation for five minutes at 1,000 x g. All samples were diluted 50-fold prior to HPLC analysis.

At low pH (≤ 8.0), solid suspensions of Compound C1 in *m*-APBA and in ammonium bicarbonate were visually identical. Both were opaque and contained varying amounts of solid, insoluble material. However, when the pH was adjusted to 9.0 dramatic differences in appearance between the two samples was observed within approximately 30 minutes. The aqueous ammonium bicarbonate control was beige in color and completely opaque; whereas, the sample containing *m*-APBA turned a transparent, dark brown. When the mixtures were subjected to centrifugation for 5 minutes at 16,000 x g and the resulting supernatants quantified by HPLC analysis, the concentration of C1 present in the *m*-APBA supernatant was 23.76 mg/ml (94% of that in the original suspension) and only 2.27 mg/ml (10% of the original suspension) was present in the ammonium bicarbonate control supernatant.

Replacement of *m*-APBA with either p-carboxyphenyl boronic acid or boric acid provided a boronate or borate complex, respectively, having increased solubility as well.

### Example 2

### Stability of Compound C1 in m-APBA at Alkaline pH

Compound C1 was suspended at 100 mg/ml (65% potency: 65 mg/ml (57 mM) in either 250 mM *m*-APBA or water (control). Separate samples were adjusted to pH 8.0, 8.5, 9.0, 9.5 and 10.0. Aliquots for time-point analysis were removed and diluted 100-fold in 100 mM acetic acid/methanol prior to HPLC injection. Stability was assessed by analysis of main peak purity, since degradation products are known to elute within the HPLC chromatographic window.

Compound C1 exhibited enhanced chemical stability in the presence of *m*-APBA in comparison to the aqueous control sample. Not only was the overall purity of the C1 sample augmented in comparison to the control, but degradation also occurred more slowly. After 9 hours at pH 9.5, the purity of the aqueous control was less than 70% of the total peak area; whereas, the sample containing *m*-APBA was in excess of 70% purity after 17 hours.

Replacement of *m*-APBA with boric acid provided a very stable borate complex. When p-carboxyphenylboronic acid was used, the complex did not appear to be as stable as the other two complexes. It is believed that the cyclic peptide ring may have partially fragmented during the complexation process due to the higher pH needed to form the complex.

## Claims

1. A reversible cyclic peptide adduct comprising a boric or boronic acid complexed with a cyclic Echinocandin anti-fungal peptide having at least one 1,2-*cis*-diol moiety wherein said adduct is ionic in character and has a tetrahedral geometry at the boron atom and is more water-soluble than said cyclic peptide having at least one 1,2-*cis*-diol moiety.

2. The reversible adduct of claim 1 wherein the adduct comprises a boric or boronic acid complexed with a cyclic Echinocandin anti-fungal peptide having the following structure C1:

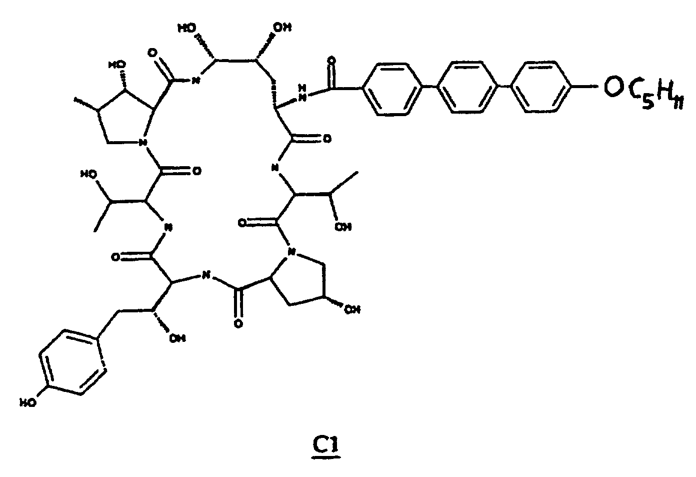

3. A method for forming a reversible cyclic peptide adduct as defined in claim 1, comprising the steps of
(i) providing an aqueous solution of a boric or boronic acid,
(ii) adding a cyclic Echinocandin anti-fungal peptide compound having at least one 1,2-*cis*-diol moiety to said aqueous solution, and
(iii) adjusting the pH of said aqueous solution to a value sufficient to effect complexation between said boric or boronic acid and said cyclic Echinocandin anti-fungal peptide compound under alkaline conditions.

4. The method of claim 3 wherein said cyclic Echinocandin anti-fungal peptide has the following structure C1:

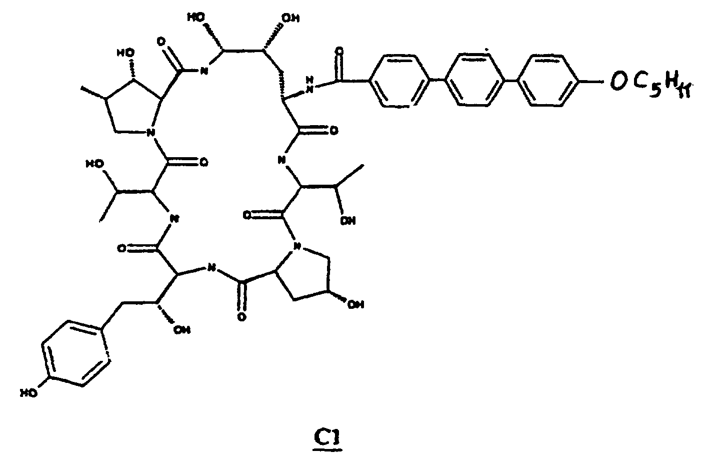

5. The adduct of claim 1 or 2 wherein said boronic acid is selected from the group consisting of alkylboronic acids, heterocycloalkyl boronic acids, arylboronic acids, and heteroarylboronic acids.

6. The adduct of claim 1 or 2 wherein said boronic acid is selected from the group consisting of ethylboronic acid, propylboronic acid, butylboronic acid, tetrahydrofuranylboronic acid, phenylboronic acid, *o*-methylphenylboronic acid, *m*-aminophenylboronic acid, *p*-methylphenylboronic acid, *p*-carboxyphenylboronic acid, [*o*-(diisopropylamino)carbonyl]phenylboronic acid, *o*-formylphenylboronic acid, *m*-formylphenylboronic acid, *p*-methoxyphenylboronic acid, *p*-nitrophenylboronic acid, *p*-fluorophenylboronic acid, p-bromophenylboronic acid, *p*-trifluoromethylphenylboronic acid, 4,4'-diphenyldiboronic acid, 1-naphthylboronic acid, thiophene-2-boronic acid, thiophene-3-boronic acid, 2-formylihiophene-2-boronic acid, 5-chlorothiophene-2-boronic acid, 5-acetylthiophene-2-boronic acid, benzo[b]thiophene-2-boronic acid, benzo[b]furan-2-boronic acid, indole-5-boronic acid.

7. The method of claim 3 or 4 wherein said boronic acid is as defined in claim 5.

8. The method of claim 3 or 4 wherein said boronic acid is as defined in claim 6.

9. The method of any of claims 3, 4, 7 and 8 wherein said aqueous solution is adjusted to a pH value between 7.5 and 9.5.

10. A method for purifying a cyclic Echinocandin anti-fungal peptide having a 1,2-*cis*-diol moiety, comprising in the following order the steps of
(i) providing a crude mixture of a cyclic Echinocandin anti-fungal peptide having at least one 1,2-*cis*-diol functionality,
(ii) complexing said at least one 1,2-*cis*-diol functionality of said cyclic Echinocandin anti-fungal peptide with a boric or boronic acid to form a reversible adduct as defined in any one of claims 1, 2, 5 and 6,
(iii) solubilizing said reversible adduct in an aqueous solution,
(iv) either
(a) removing any insoluble materials from said aqueous solution or
(b) concentrating said aqueous solution to form a concentrate, absorbing said concentrate onto a reverse-phase hydrophobic resin packed in a chromatography column, eluting with an aqueous solvent system, and combining effluent fractions containing said reversible adduct into a single effluent solution,
(v) acidifying said aqueous solution to a pH value equal to or less than the pKa of said boric or boronic acid to decomplex said reversible adduct, and
(vi) recovering said cyclic Echinocandin anti-fungal peptide from said aqueous solution.

11. A pharmaceutical formulation comprising a reversible adduct as defined in any one of claims 1, 2, 5 and 6.

12. The pharmaceutical formulation of claim 11 further comprising a pharmaceutically inert carrier, a wetting agent, lubricating agent, emulsifier, suspending agent, preservative, sweetener, stabilizer, perfuming agent, flavouring agent or combinations thereof.

13. The pharmaceutical formulation of claim 12 wherein said inert carrier is water.

14. An adduct according to any of claims 1, 2, 5 and 6 for use in the treatment of fungal infection.

15. Use of an adduct according to any of claims 1, 2, 5 and 6 for the manufacture of a medicament for the treatment of fungal infection.

## Patentansprüche

1. Reversibles cyclisches Peptid-Addukt, welches eine Bor- oder Boronsäure, komplexiert mit einem cyclischen fungiziden Echinocandin-Peptid mit mindestens einer 1,2-*cis*-Diolkomponente, umfasst, wobei das Addukt von ionischem Charakter ist und eine tetrahedrale Geometrie am Boratom aufweist und wasserlöslicher ist als das cyclische Peptid mit mindestens einer 1,2-*cis*-Diolkomponente.

2. Reversibles Addukt nach Anspruch 1, wobei das Addukt eine Bor- oder Boronsäure, komplexiert mit einem cyclischen fungiziden Echinocandin-Peptid, mit der folgenden Struktur C1 umfasst:

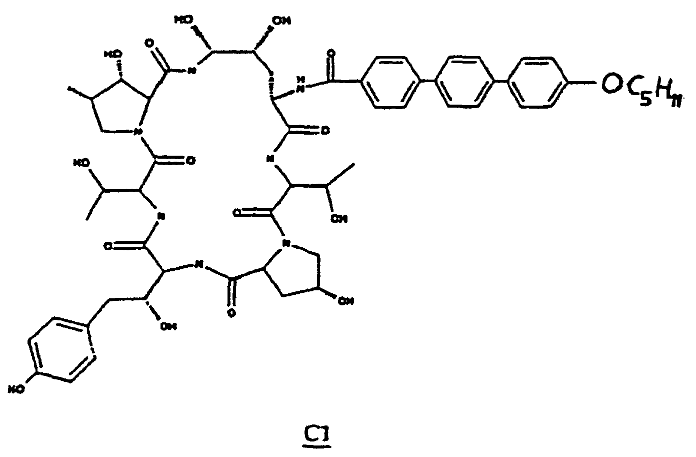

3. Verfahren zur Herstellung eines reversiblen cyclischen Peptid-Addukts, wie in Anspruch 1 definiert, welches die folgenden Schritte umfasst:
(i) Herstellen einer wässrigen Lösung einer Bor- oder Boronsäure,
(ii) Hinzufügen einer cyclischen fungiziden Echinocandin-Peptidverbindung mit mindestens einer 1,2-*cis*-Diolkomponente zur wässrigen Lösung, und
(iii) Einstellen des pH-Werts der wässrigen Lösung auf einen geeigneten Wert, um die Komplexbildung zwischen der Bor- oder Boronsäure und der cyclischen fungiziden Echinocandin-Peptidverbindung unter alkalischen Bedingungen zu bewirken.

4. Verfahren nach Anspruch 3, wobei das cydische fungizide Echinocandin-Peptid die folgende Struktur C1 aufweist:

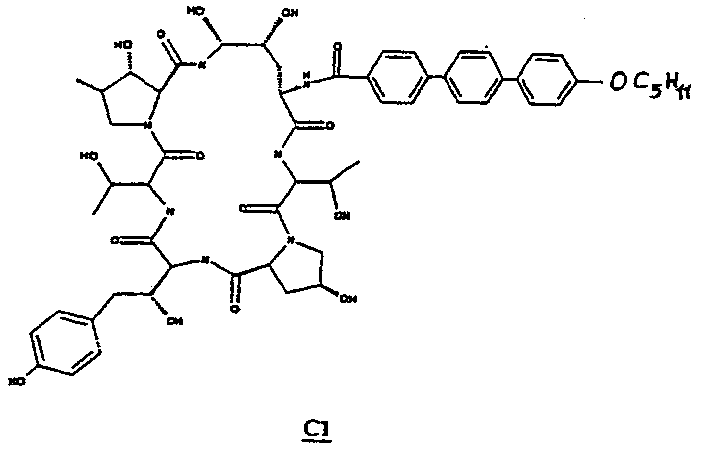

5. Addukt nach Anspruch 1 oder 2, wobei die Boronsäure gewählt ist aus der Gruppe, bestehend aus Alkylboronsäuren, Heterocycloalkylboronsäuren, Arylboronsäuren und Heteroarylboronsäuren.

6. Addukt nach Anspruch 1 oder 2, wobei die Boronsäure gewählt ist aus der Gruppe, bestehend aus Ethylboronsäure, Propylboronsäure, Butylboronsäure, Tetrahydrofuranylboronsäure, Phenylboronsäure, *o*-Methylphenylboronsäure, *m*-Aminophenylboronsäure, *p*-Methylphenylboronsäure, *p*-Carboxyphenylboronsäure, [*o*-(Diisopropylamino)carbonyl]phenylboronsäure, *o*-Formylphenylboronsäure, *m*-Formylphenylboronsäure, *p*-Methoxyphenylboronsäure, *p*-Nitrophenylboronsäure, *p*-Fluorphenylboronsäure, *p*-Bromphenylboronsäure, *p*-Trifluormethylphenylboronsäure, 4,4'-Diphenyldiboronsäure, 1-Naphthylboronsäure, Thiophen-2-boronsäure, Thiophen-3-boronsäure, 2-Formylthiophen-2-boronsäure, 5-Chlorthiophen-2-boronsäure, 5-Acetylthiophen-2-boronsäure, Benzo[b]thiophen-2-boronsäure, Benzo[b]furan-2-boronsäure, Indol-5-boronsäure.

7. Verfahren nach Anspruch 3 oder 4, wobei die Boronsäure wie in Anspruch 5 definiert ist.

8. Verfahren nach Anspruch 3 oder 4, wobei die Boronsäure wie in Anspruch 6 definiert ist.

9. Verfahren nach einem der Ansprüche 3, 4, 7 und 8, wobei die wässrige Lösung auf einen pH-Wert von zwischen 7,5 und 9,5 eingestellt ist.

10. Verfahren zum Reinigen eines cyclischen fungiziden Echinocandin-Peptids mit einer 1,2-*cis*-Diolkomponente, welches die folgenden Schritte in dieser Reihenfolge umfasst:
(i) Herstellen eines Rohgemischs aus einem cyclischen fungiziden Echinocandin-Peptid mit mindestens einer 1,2-*cis*-Diol-Funktionalität,
(ii) Komplexieren der mindestens einen 1,2-*cis*-Diol-Funktionalität und des cyclischen fungiziden Echinocandin-Peptids mit einer Bor- oder Boronsäure zum Erhalt eines reversiblen Addukts, wie in einem der Ansprüche 1, 2, 5 und 6 definiert,
(iii) Anlösen des reversiblen Addukts in einer wässrigen Lösung,
(iv) entweder
(a) Entfernen jeglichen unlöslichen Materials aus der wässrigen Lösung oder
(b) Konzentrieren der wässrigen Lösung zum Erhalt eines Konzentrats, Absorbieren des Konzentrats auf einem in eine Chromatographiesäule gepackten hydrophoben Umkehrphasen-Harz, Eluieren mit einem wässrigen Lösungsmittelsystem und Kombinieren der ablaufenden Fraktionen, die das reversible Addukt enthalten, zu einer einzigen Ablauflösung,
(v) Ansäuern der wässrigen Lösung auf einen pH-Wert von gleich oder weniger als dem pKa der Bor- oder Boronsäure, um das reversible Addukt zu dekomplexieren, und
(vi) Rückgewinnen des cyclischen fungiziden Echinocandin-Peptids aus der wässrigen Lösung.

11. Pharmazeutische Formulierung, welche ein reversibles Addukt, wie in einem der Ansprüche 1, 2, 5 und 6 definiert, umfasst.

12. Pharmazeutische Formulierung nach Anspruch 11, welche außerdem einen pharmazeutisch inerten Träger, ein Benetzungsmittel, Schmiermittel, Emulgator, Suspendiermittel, Konservierungsmittel, Süßstoff, Stabilisator, Duftstoff, Geschmacksstoff oder Kombinationen davon umfasst.

13. Pharmazeutische Formulierung nach Anspruch 12, wobei der inerte Träger Wasser ist.

14. Addukt nach einem der Ansprüche 1, 2, 5 und 6, zur Verwendung bei der Behandlung einer Pilzinfektion.

15. Verwendung eines Addukts nach einem der Ansprüche 1, 2, 5 und 6, zur Herstellung eines Medikaments für die Behandlung einer Pilzinfektion.

## Revendications

1. Produit d'addition de peptide cyclique réversible comprenant un acide borique ou boronique complexé avec un peptide antifongique cyclique Echinocandine ayant au moins un groupe 1,2-*cis*-diol, ledit produit d'addition étant de caractère ionique et ayant une géométrie tétraédrique à l'atome de bore et étant plus hydrosoluble que ledit peptide cyclique ayant au moins un groupe 1,2-*cis*-diol.

2. Produit d'addition réversible selon la revendication 1, dans lequel le produit d'addition comprend un acide borique ou boronique complexé avec un peptide antifongique cyclique Echinocandine ayant la structure C1 suivante :

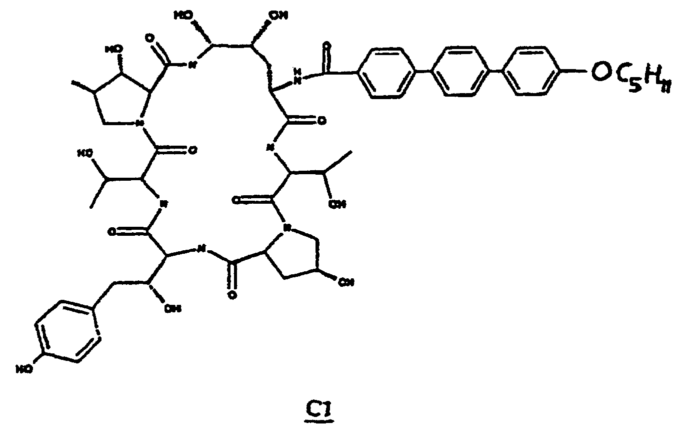

3. Procédé de formation d'un produit d'addition de peptide cyclique réversible tel que défini dans la revendication 1, comprenant les étapes consistant
(i) à fournir une solution aqueuse d'un acide borique ou boronique,
(ii) à ajouter un composé peptidique antifongique cyclique Echinocandine ayant au moins un groupe 1,2-*cis*-diol à ladite solution aqueuse, et
(iii) à ajuster le pH de ladite solution aqueuse à une valeur suffisante pour effectuer une complexation entre ledit acide borique ou boronique et ledit composé peptidique antifongique cyclique Echinocandine dans des conditions alcalines.

4. Procédé selon la revendication 3, dans lequel ledit peptide antifongique cyclique Echinocandine a la structure C1 suivante :

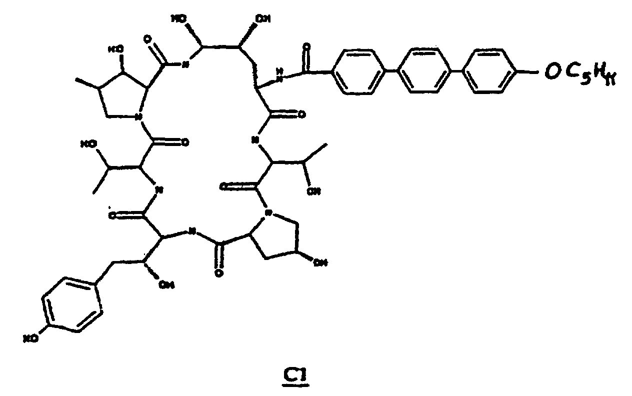

5. Produit d'addition selon la revendication 1 ou 2, dans lequel ledit acide boronique est choisi dans le groupe constitué par les acides alkylboroniques, les acides hétérocycloalkylboroniques, les acides arylboroniques et les acides hétéroarylboroniques.

6. Produit d'addition selon la revendication 1 ou 2, dans lequel ledit acide boronique est choisi dans le groupe constitué par l'acide éthylboronique, l'acide propyl-boronique, l'acide butylboronique, l'acide tétrahydro-furanylboronique, l'acide phénylboronique, l'acide *o*-méthylphénylboronique, l'acide *m*-aminophénylboronique, l'acide *p*-méthylphénylboronique, l'acide *p*-carboxyphényl-boronique, l'acide [*o*-(diisopropylamino)carbonyl]phényl-boronique, l'acide *o*-formylphénylboronique, l'acide *m*-formylphénylboronique, l'acide *p*-méthoxyphénylboronique, l'acide *p*-nitrophénylboronique, l'acide *p*-fluorophényl-boronique, l'acide *p*-bromophénylboronique, l'acide *p*-trifluorométhylphénylboronique, l'acide 4,4'-diphényl-diboronique, l'acide 1-naphtylboronique, l'acide thiophène-2-boronique, l'acide thiophène-3-boronique, l'acide 2-formylthiophène-2-boronique, l'acide 5-chlorothiophène-2-boronique, l'acide 5-acétylthiophène-2-boronique, l'acide benzo[b]thiophène-2-boronique, l'acide benzo[b]furan-2-boronique, l'acide indole-5-boronique.

7. Procédé selon la revendication 3 ou 4, dans lequel ledit acide boronique est tel que défini dans la revendication 5.

8. Procédé selon la revendication 3 ou 4, dans lequel ledit acide boronique est tel que défini dans la revendication 6.

9. Procédé selon l'une quelconque des revendications 3, 4, 7 et 8, dans lequel ladite solution aqueuse est ajustée à une valeur de pH entre 7,5 et 9,5.

10. Procédé pour purifier un peptide antifongique cyclique Echinocandine ayant un groupe 1,2-*cis*-diol, comprenant, dans l'ordre suivant, les étapes consistant
(i) à fournir un mélange brut d'un peptide antifongique cyclique Echinocandine ayant au moins une fonctionnalité 1,2-*cis*-diol,
(ii) à complexer ladite au moins une fonctionnalité 1,2-*cis*-diol dudit peptide antifongique cyclique Echinocandine avec un acide borique ou boronique pour former un produit d'addition réversible tel que défini dans l'une quelconque des revendications 1, 2, 5 et 6,
(iii) à solubiliser ledit produit d'addition réversible dans une solution aqueuse,
(iv) soit
(a) à éliminer toutes matières insolubles de ladite solution aqueuse; soit
(b) à concentrer ladite solution aqueuse pour former un concentré, à absorber ledit concentré sur une résine hydrophobe en phase inverse tassée dans une colonne de chromatographie, en éluant avec un système solvant aqueux, et à combiner les fractions d'effluent contenant ledit produit d'addition réversible en une seule solution d'effluent,
(v) à acidifier ladite solution aqueuse à une valeur de pH égale ou inférieure au pKa dudit acide borique ou boronique pour décomplexer ledit produit d'addition réversible, et
(vi) à récupérer ledit peptide antifongique cyclique Echinocandine à partir de ladite solution aqueuse.

11. Formulation pharmaceutique comprenant un produit d'addition réversible tel que défini dans l'une quelconque des revendications 1, 2, 5 et 6.

12. Formulation pharmaceutique selon la revendication 11, comprenant en outre un véhicule pharmaceutiquement inerte, un agent mouillant, un agent lubrifiant, un émulsifiant, un agent de suspension, un conservateur, un édulcorant, un stabilisant, un agent parfumant, un agent aromatisant ou des combinaisons de ceux-ci.

13. Formulation pharmaceutique selon la revendication 12, dans laquelle ledit véhicule inerte est l'eau.

14. Produit d'addition selon l'une quelconque des revendications 1, 2, 5 et 6 pour l'utilisation dans le traitement d'une infection fongique.

15. Utilisation d'un produit d'addition selon l'une quelconque des revendications 1, 2, 5 et 6 pour la fabrication d'un médicament pour le traitement d'une infection fongique.
